# EUROPEAN PATENT SPECIFICATION

(11) **EP 4 494 693 B1**
(45) Date of publication and mention of the grant of the patent: **18.11.2026**
(21) Application number: 24188973.2
(22) Date of filing: 16.07.2024
(51) Int. Cl.: A61N 1/39, A61B 5/361, H01Q 1/22, A61N 1/04

(54) **ANTENNA MOUNTS FOR MEDICAL DEVICES**
ANTENNENHALTERUNGEN FÜR MEDIZINISCHE VORRICHTUNGEN
SUPPORTS D'ANTENNE POUR DISPOSITIFS MÉDICAUX

(30) Priority: 17.07.2023 US 202363513981 P
(43) Date of publication of application: 22.01.2025
(73) Proprietor: Stryker Corporation, Portage, MI 49002-9711 (US)
(72) Inventor: SOHN, Dennis Changmin, Kalamazoo, MI 49002 (US); CHESTER, Steven M., Kalamazoo, MI 49002 (US); CHOI, Kyujin, Kalamazoo, MI 49002 (US)
(74) Representative: Kilburn & Strode LLP

(56) References cited:
- US-A1- 2015 138 024
- US-A1- 2020 280 120
- US-B2- 9 902 028

## Description

This application relates to a defibrillator device, comprising:
a housing;
an antenna mount disposed on an interior surface of the housing and comprising a material that is flexible and electrically insulative, wherein a hole extends through the antenna mount; and
a flexible antenna disposed between the interior surface of the housing and the antenna mount. The application further relates to a method of manufacturing such a device.

### BACKGROUND

Medical devices and other portable devices may have wireless communication capabilities to transfer information to external devices over wireless networks. Wireless networks include cellular networks, wireless local area networks (WLANs), and other networks utilizing radio communications. Many medical devices are used in non-clinical settings, such as at the scene of an emergency, at a home, at an airport, etc. Wireless connectivity enables a device to transmit patient information to a care provider, such as a physician, for remote guidance of medical care. For devices stored in non-clinical settings, wireless connectivity can enable remote troubleshooting and data storage.

To implement wireless connectivity, portable devices generally include an antenna to transmit and receive electromagnetic (EM) signals more effectively. Antennas can transmit EM signals indicative of physiological parameters detected from a patient, treatments administered to the patient, or other information detected or generated by a medical device. Antennas can also receive EM signals indicative of communications from a care provider.

Minimizing device size can improve the ease of use of portable devices. The development of flexible antennas can enable wireless communication in portable devices with size and/or shape limitations.

US 2015/0138024 discloses an antenna placed inside a housing of portable device, such as a smartphone. The antenna comprises a flexible substrate on which a conductive radiator pattern is provided, for instance by flexible printed circuit (FPC) technology or laser direct structuring (LDS) technology. The flexible substrate conforms to the interior of the housing.

### BRIEF DESCRIPTION OF THE DRAWINGS

FIG. 1 illustrates an environment in which a defibrillator is monitoring a patient and communicating with an external device.
FIG. 2 illustrates an example antenna mount disposed on an interior surface of a device housing.
FIG. 3 illustrates an example antenna mount bending at a radius of curvature.
FIG. 4 illustrates an example antenna mount with antenna diversity and dynamic antenna positioning.
FIG. 5 illustrates an example antenna mount attached to a dampener and a dampener.
FIG. 6 illustrates an example process for integrating an antenna into a device.
FIG. 7 illustrates an example process for manufacturing an antenna mount and integrating an antenna into a device.
FIG. 8 illustrates an example of an external defibrillator configured to perform various functions described herein.

### DETAILED DESCRIPTION

The invention is defined by the independent claims. Various implementations described herein relate to apparatuses, methods, and systems for installing an antenna in a device, such as a medical device. According to various cases, an antenna mount is utilized to mount an antenna on an interior wall of a medical device housing. In particular examples, the antenna mount is flexible, which enables it to conform to the interior wall, regardless of the shape of the interior wall. Accordingly, the antenna mount may be utilized to install an antenna in the medical device well after the medical device was designed and manufactured. In various examples, a hole extending through the antenna mount accommodates one or more wires extending from the antenna to internal circuitry of the medical device. An edge of the hole, for instance, includes an electrically insulative material that prevents interference of electrical signals conducted through the one or more wires. In particular instances, the antenna mount may be utilized to install a wireless charging coil in the medical device, thereby enabling the medical device to develop wireless charging capabilities during its product lifetime.

Implementations of the present disclosure will now be described with reference to the accompanying figures.

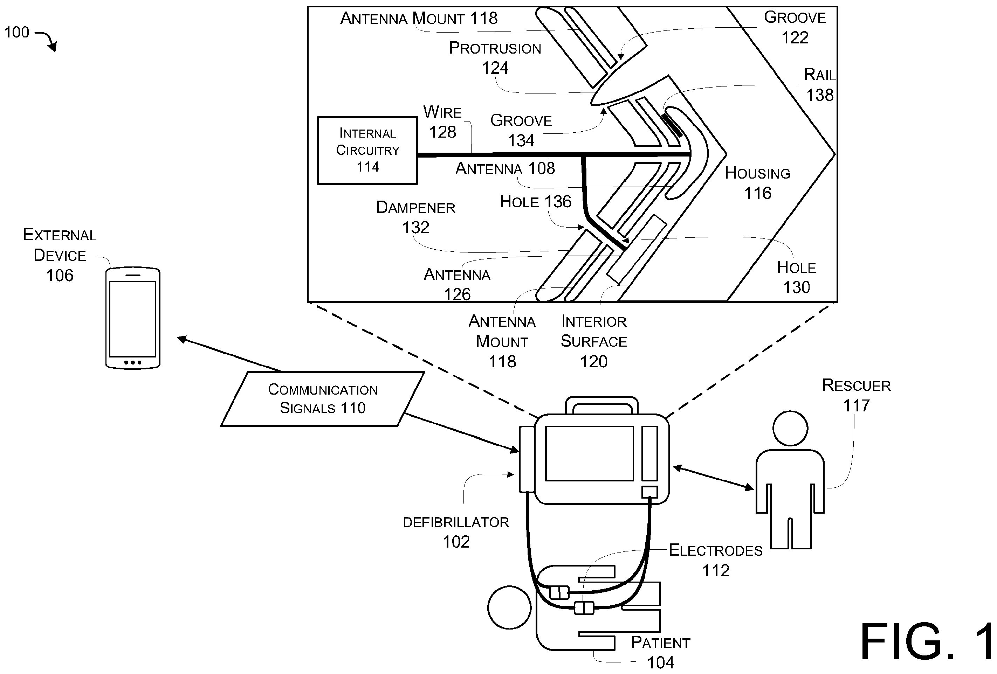

FIG. 1 illustrates an environment 100 in which a defibrillator 102 is monitoring a patient 104 and communicating with an external device 106. The defibrillator 102, in various examples, is a monitor-defibrillator or an automated external defibrillator. The defibrillator 102 may be configured for professional use (e.g., by a trained user, an emergency responder, etc.) or for public use (e.g., by an untrained user, a bystander etc.). The defibrillator 102 includes an antenna 108 configured to transmit and receive communication signals 110. In this example, the patient 104 is experiencing cardiac arrest.

The defibrillator 102 is a medical device configured to monitor a heart of the patient 104 and output an electrical shock to the patient 104. In various implementations, the defibrillator 102 includes electrodes 112, internal circuitry 114, and a housing 116. In some implementations, the electrodes 112 are removably connected to the defibrillator 102 and are disposed on different locations on the patient 104. In some cases, the electrodes 112 are configured to detect an electrical signal representative of the electrical activity of the heart of the patient 104. The internal circuitry 114, for example, includes a measurement circuit configured to detect the ECG (e.g., a 5 lead ECG, a 12 lead ECG, etc.) of the patient 104 based on the electrical signal detected by the electrodes 112.

According to some implementations, the defibrillator 102 is configured to deliver an electrical shock to the patient 104 via the electrodes 112. The internal circuitry 114, for instance, includes a treatment circuit configured to output an electrical shock the patient 104 using the electrodes 112. In some cases, the internal circuitry 114 includes a processor configured to perform operations. In some cases, the internal circuitry 114 includes memory that is connected to the processor. In various implementations, the memory stores instructions that, when executed by the processor, causes the processor to perform various operations. Using the internal circuitry 114, the defibrillator 102, for instance, is configured to analyze the ECG. In some examples, the defibrillator 102 is configured to determine, based on the ECG, that the patient 104 is exhibiting ventricular fibrillation (VF). According to some examples, in response to determining that the patient is exhibiting VF, to the defibrillator 102 outputs the electrical shock to the patient 104.

In various implementations, the defibrillator 102 includes a housing 116 that at least partially encloses other elements of the defibrillator 102. For example, the housing 116 encloses the internal circuitry 114, the processor, the memory, or any combination thereof. In various examples, the housing 116 acts as a barrier to moisture, electrical interference, and/or dust, thereby protecting various components in the defibrillator 102 from damage. The housing 116 may include a polymer (e.g., acrylic, polycarbonate, copolyester, etc.), a metal (e.g., aluminum, stainless steel, etc.), or the like.

In some examples, the defibrillator 102 is a portable medical device configured to be operated outside of a clinical environment by a rescuer 117. For instance, the environment 100 could be at the scene of a car crash, in an airport terminal, in a residence, or some other place in which the patient 104 is experiencing a sudden medical emergency. In some cases, a rescuer 117 brings the defibrillator 102 to the patient 104 in response to a report that the patient 104 has lost consciousness. The rescuer 117 is, in some examples, an untrained user, such as a bystander, or any other person present when the patient 104 experiences cardiac arrest. The defibrillator 102, for instance, may monitor and/or treat the patient 104 in the environment 100 before the patient 104 is transferred to a clinical care environment, such as a hospital, for further care.

In some cases, it may be advantageous for the defibrillator 102 to communicate with the external device 106. The external device 106, for example, is a device configured to communicate with the defibrillator 102 to assist with the care of patient 104. Examples of the external device 106 include a device operated by a care provider (e.g., a physician, emergency medical technician (EMT), a paramedic, or a lay user), a device configured to process ECG signals, a device configured to assist with monitoring or treatment of the patient 104, or any other device related to medical care. The external device 106 is, in some cases, a mobile device, an Internet of Things (IoT) device, a computer (e.g., a laptop device, a server, etc.), or any other network device configured to communicate over a communication network.

In various cases, the external device 106 includes wireless communication capabilities. For example, the external device 106 may be located remotely from the defibrillator 102, such as at a remote clinical environment (e.g., a hospital). In some cases, the external device 106 is a computing device utilized by a care provider (e.g., a physician) at the downstream clinical care environment. One or more physiological parameters of the patient 104 detected by the defibrillator 102 may be relevant to an eventual diagnosis by the care provider. Further, one or more treatments administered to the patient 104 by the defibrillator 102 in the environment 100 may be relevant to the administration of further treatments in the clinical care environment. Thus, the care of the patient 104 can be enhanced if the defibrillator 102 is configured to communicate data indicative of physiological parameters, treatments, and other relevant information to the external device 106. Due to the portability requirements of the defibrillator 102, it is advantageous that such communication is performed wirelessly.

In various implementations, the defibrillator 102 communicates wirelessly with the external device 106 using the antenna 108. The antenna 108 may have any suitable structure for receiving wireless communications and for conforming to the housing 116 of the device. In some instances, the antenna 108 is an internal antenna, such as a microstrip antenna, a helical antenna, a planar inverted-F antenna, or another suitable antenna. For example, the antenna 108 is a microstrip radio frequency identification (RFID) tag antenna that communicates communication signals 110 by receiving, modifying, and reflecting radio frequency energy that is transmitted by a RFID reader antenna attached to the external device 106. In some instances, the antenna 108 is a wire antenna (such as a dipole antenna, a monopole antenna, a loop antenna, etc.). The antenna 108, in some cases, is connected to an external antenna. In some instances, the antenna 108 is a flexible antenna.

According to various implementations, the antenna 108 is configured to transmit and receive communication signals 110 using one or more wireless networks. Examples of wireless networks include WI-FI^{®}, cellular networks, wireless local area networks (WLANs), and BLUETOOTH^{®}. In some instances, the communication signals 110 are electromagnetic (EM) signals, radio waves, or the like. In some examples, the antenna 108 transmits radio waves to the external device 106 via a cell tower. In some cases, the antenna 108 is connected to a wireless modem in the defibrillator 102, such as a modem for engaging in WI-FI^{®}, WIGIG^{®}, WIMAX^{®}, BLUETOOTH^{®}, or infrared communication. In some cases, the antenna 108 is connected to a component in the defibrillator 102 that enable use of a communication network, such as a network interface card (NIC), a network adapter, a local area network (LAN) adapter, or a physical, virtual, or logical address to connect to the various external devices and/or systems.

Using the antenna 108, the defibrillator 102 transmits communication signals 110 to and/or receives communication signals 110 from the external device 106. In some examples, the communication signals 110 are indicative of ECG data, electrical signals detected from the patient 104, another physiological parameter of the patient 104, or treatment event information. Treatment events, for instance, include electrical shocks or pace pulses administered to the patient 104 by the defibrillator 102. In various implementations, using the internal circuitry 114, the defibrillator 102 is configured to generate ECG data based on the electrical signals detected from the patient 104. According to various cases, the internal circuitry 114 causes the antenna 108 to transmit the communication signals 110 indicative of the ECG data to the external device 106. In some examples, the communication signals 110 are transmitted to the external device 106 for patient monitoring and/or signal processing. In some implementations, the antenna 108 receives communication signals 110 from the external device 106, and the antenna 108 provides the received communication signals 110 to the internal circuitry 114 for processing. In some examples, the antenna 108 receives communication signals 110 indicative of guidance from a care provider operating the external device 106. For example, the care provider may communicate, using the external device 106, to the defibrillator 102 that the patient 104 requires cardiopulmonary resuscitation (CPR). In some instances, the defibrillator 102 is configured to transmit communication signals 110 on a schedule, upon input of a user (e.g., the rescuer 117), or based on treatment events. For example, the defibrillator 102 may be configured to transmit communication signals 110 during a time in which the defibrillator 102 is not administering a treatment event.

In some cases, it may be beneficial to integrate the antenna 108 into the existing housing 116 of the defibrillator 102. For example, the antenna 108 may utilize a technology that was developed after regulatory approval and marketing for the defibrillator 102 was achieved. A defibrillator 102, for instance, may be marketed for years or even decades after its initial approval, due to the substantial hurdles in designing new medical devices and for obtaining regulatory approval of new medical devices. Therefore, it may be advantageous to retrofit the existing defibrillator 102 for use with a new antenna 108, rather than developing an entirely new model of the defibrillator 102 each time the new antenna 108 is developed. However, in various examples, the housing 116 and other structural features of the defibrillator 102 may not have been specifically designed for use with the antenna 108. Thus, it may be challenging to retrofit the defibrillator 102 for use with the antenna 108.

Implementations of the present disclosure address these and other problems by enabling different types of antennas to be installed into the housing of existing medical devices, such as the defibrillator 102. Using the techniques described herein, the antenna 108 can be installed within the defibrillator 102, even if the defibrillator 102 was designed long before the development of the antenna 108. For example, the antenna 108 may be installed in the defibrillator 102 in a functional and physically stable fashion, even when a housing 116 of the defibrillator 102 is not specifically designed to accommodate the antenna 108.

These issues can be addressed, for example, by attached the antenna 108 to an antenna mount 118 that can be easily inserted into the defibrillator 102. According to various implementations, the antenna mount 118 includes a flexible substrate 119 that can conform to the existing housing 116 of the defibrillator 102. In various cases, the flexible substrate 119 is flexible and electrically insulative. The flexible substrate 119 is configured to conform to an interior surface 120 of the housing 116 of the defibrillator 102. In some implementations, the flexible substrate 119 has a thickness between 0.1 millimeters (mm) and 3 mm. In some implementations, the flexible substrate 119 can bend at a radius of curvature of at most 1 centimeter (cm) without breaking. For example, the flexible substrate 119 can fold over on itself, bending at a radius of curvature of 0.15 cm. The flexible substrate 119 includes an electrically insulative material. According to some implementations, the electrically insulative material is a polymer, such as polycarbonate, polyethylene, polypropylene, acrylic, or aramid paper. In some examples, the antenna 108 attaches to the electrically insulative material. According to various implementations, the flexible substrate 119 is formed using cutting processes. In some examples, the flexible substrate 119 is formed by laser cutting, waterjet cutting, die cutting, or stamping.

According to some implementations, the antenna mount 118 is disposed on the interior surface 120 of the housing 116 of the defibrillator 102 in a physically stable fashion. In various examples, movement of the antenna mount 118 on the interior surface 120 is constrained. In some cases, a height of the flexible substrate 119 is equal to or less than a height of the housing 116 of the defibrillator 102. For example, the height of the flexible substrate 119 may be equal to the height of the housing 116. In some implementations, the antenna mount 118 is removably attached to the interior surface 120 of the housing 116 of the defibrillator 102 with an adhesive. In some examples, the adhesive includes pressure sensitive adhesive (PSA), epoxy resin, acrylic resin, hot melt glue, casein, double-sided tape, glue mounts, or flat adhesive pads. The antenna mount 118 is, in some cases, easily removable from the defibrillator 102. According to various implementations, the housing 116 of the defibrillator 102 includes a protrusion 124. The protrusion 124 is configured, for example, to fit inside a groove 122 in the flexible substrate. In some examples, the protrusion 124 is a rib, a notch, a latch, or any other structure disposed on the housing 116 that extends into the interior of the defibrillator 102.

According to various implementations, the antenna 108 is disposed between the antenna mount 118 and the interior surface of the housing 116 of the defibrillator 102. In some examples, the antenna 108 is attached to the antenna mount 118 with an adhesive, such as PSA, epoxy resin, acrylic resin, hot melt glue, casein, double-sided tape, glue mounts, or flat adhesive pads. In some cases, the antenna 108 is configured to be disposed in a sleeve or cutout in the antenna mount 118. The cutout may include a hole, a slit, or the like. For instance, the antenna 108 may be routed through one or more cutout(s) through the flexible substrate 119. The antenna 108 may be removable from the antenna mount 118. The antenna 108 is attached to the antenna mount 118 in a vertical orientation, horizontal orientation, or any other suitable orientation. In some implementations, the position of the antenna 108 is determined to improve transmission of the communication signals 110. In some cases, the antenna 108 is physically separated from the internal circuitry 114 of the defibrillator 102. In some cases, the antenna 108 is positioned perpendicular to a printed circuit board (PCB) in the defibrillator 102. For instance, the internal circuitry 114 may be mounted on the PCB. In some implementations, the antenna 108 is a flexible antenna and is configured to conform to the antenna mount 118.

In some examples, the antenna 108 may be attached to the antenna mount 118 using a magnet. For instance, the antenna 108 and the antenna mount 118 may each include or be attached to a magnetic material. In some examples, the antenna 108 includes or is attached to a ferromagnetic material, and the antenna mount 118 includes a magnet, such as a permanent rare earth magnet or an electromagnet. In some examples, the antenna 108 includes or is attached to the magnet, and the antenna mount 118 includes the ferromagnetic material. The ferromagnetic material may include steel, iron, nickel, cobalt, steel, or the like. The permanent rare earth magnet may include a neodymium alloy, a samarium alloy, a dysprosium alloy, or the like. The electromagnet, in various examples, includes a metal core (e.g., steel, iron, rare earth magnet, or the like) wrapped by a wire coil (e.g., copper wire, aluminum wire, or the like). According to various implementations, the antenna 108 may be attached to the antenna mount 118 using at least one of: an adhesive, a magnet, a cutout, or any other suitable mechanism.

In various instances, the antenna mount 118 enables antenna diversity, including spatial diversity, polarization diversity, frequency diversity, pattern diversity, directional diversity, time diversity, and transmit-receive diversity. In some examples, an antenna 126 is attached to the antenna mount 118 and positioned at a distance from antenna 108 for spatial diversity. In some examples, the antenna 126 has a different radiation pattern than antenna 108 for pattern diversity. In some cases, the antenna 126 has orthogonal polarization to the antenna 108 for polarization diversity.

According to various implementations, a wire 128 carries the communication signals 110 from the internal circuitry 114 to the antenna 108 for transmission to the external device 106. According the various implementations, the wire 128 is routed through a hole 130 through the flexible substrate 119 to the internal circuitry of the defibrillator 102. In some examples, an edge of the hole includes the electrically insulative material of the flexible substrate 119. As used herein, the term "edge of a hole," and its equivalents refers to the curved surface which extends through the antenna mount and represents the perimeter of the hole. In some implementations, the wire 128 is attached to the antenna mount 118 with an adhesive. The adhesive, in some examples, includes double-sided tape, glue mounts, or flat adhesive pads. In some cases, the wire 128 is routed through more than one hole through the flexible substrate, including the hole 130. According to various implementations, the position of the wire 128 is determined to reduce interference. For example, the position of the wire 128 is based on the position of other internal components in the defibrillator 102.

In some implementations, a dampener 132 is attached to the antenna mount 118 to improve transmission of communication signals 110 by the antenna 108. The dampener 132 may reduce noise in the communication signals 110 or data encoded in the communication signals 110 by reducing physical vibrations of the antenna 108 and antenna mount 118. In various cases, the dampener 132 is configured to vibrate or otherwise move in order to absorb and dissipate kinetic energy of the defibrillator 102. The presence of the dampener 132 may therefore improve signal transmission. The dampener 132 is, in some cases, configured to conform to shape of the antenna mount 118. The dampener 132, for instance, bends at a radius of curvature of 1 cm without breaking. In some cases, the dampener 132 includes a foam or a spray foam. For example, the dampener may include polystyrene, latex, polyurethane (e.g., viscoelastic polyurethane), polyethylene, or polypropylene. The dampener 132 can be disposed between the antenna mount 118 and the internal circuitry 114. The position of the dampener 132 is, in some cases, physically stable. For example, a height of the dampener 132, for instance, is equal to or less than a height of the housing 116 to constrain vertical movement of the dampener. In some examples, a groove 134 is disposed through the dampener 132. The protrusion 124 in the housing is configured to fit in the groove 134. In some embodiments, a hole 136 is disposed through the dampener 132. The wire 128 connected to the antenna 108 is routed through the hole 136.

In some cases, the antenna 108 attaches to a rail 138 on the antenna mount 118 to enable dynamic positioning of the antenna 108. The antenna 108 attaches to the rail 138 using a hook, a roller, a sliding block, a rack and pinion, a linear bearing, or another sliding mechanism. The rail 138 is coupled to an actuator that controls the position of the antenna 108 along the rail 138. The actuator is, in some examples, a manual crank, a linear actuator, a rotational actuator, a servo motor, a magnetic actuator, a shape memory alloy actuator, or any other rotary or linear actuator that enables dynamic positioning. In some instances, the actuator is controlled externally. According to some implementations, the actuator is positioned central to the defibrillator 102 or on a perimeter of the defibrillator 102. In some cases, the actuator is configured to update the position of the antenna 108 based on real-time radio conditions of the defibrillator 102, in order to optimize the position of the antenna 108 within the defibrillator 102. In some examples, the antenna 108 is physically coupled to the actuator. In some instances, the antenna 108 is physically coupled to an intermediary substrate (e.g., a belt) that is physically coupled to the actuator. For instance, the antenna 108 could be physically coupled to a belt that enables the antenna 108 to be moved around a perimeter of the defibrillator 102.

In some examples, the wireless communication requirements of the defibrillator 102 and the external device 106 may change over time. For example, new communication technologies are being developed at a rapid rate. In contrast to the rapid development of new communication technologies, medical devices such as the defibrillator 102 are designed to be utilized for many years, or even decades. Thus, in some cases, the antenna 108 may need to be replaced with a next-generation antenna as communication technologies evolve (e.g., 4^{th} Generation (4G) to 5^{th} Generation (5G) cellular network technologies).

Implementations of the present disclosure address these and other problems by enabling antenna mounts to be easily removed from the housing of medical devices, such as the defibrillator 102, and efficiently replaced. Using the techniques described herein, the antenna mount 118 can installed into the defibrillator 102 in a physically stable and removable fashion. A new antenna mount can be formed in a timely manner to replace the antenna mount 118 using a cutting process. The issue is addressed, for example, by installing the antenna mount 118 by positioning the groove 122 around the protrusion 124. The height of the antenna mount 118 can be equal to the height of the housing 116 to stabilize the position of the antenna mount 118.

In some examples, the antenna 108 may need to be repositioned along the housing 116 of the defibrillator 102. For example, a component of the internal circuitry 114 may be replaced and cause interference with the antenna 108. Implementations of the present disclosure address these and other problems by enabling the repositioning of the antenna 108 on the existing antenna mount 118. The issue is addressed, for example, by installing the antenna 108 onto the rail 138. The rail 138 is, in some cases, coupled to the actuator that can be controlled externally.

In some implementations, the antenna 108 can be replaced by or disposed against a wireless charging coil. In various examples, the wireless charging coil attaches to the antenna mount 118 with an adhesive. The wireless charging coil may be attached to the rail 138 on the antenna mount 118. A wire connected to the wireless charging coil, in some cases, connects to the internal circuitry 114 of the defibrillator 102 through a hole (e.g., the hole 130) through the antenna mount 118. In various implementations, the wireless charging coil generates an alternating EM field to transfer energy from a second wireless charging coil, which may be located outside of the defibrillator 102. In some instances, the wireless charging coil charges a battery, capacitor, or other power storage of the defibrillator 102 when placed next to an external wireless charger that includes a second wireless charging coil.

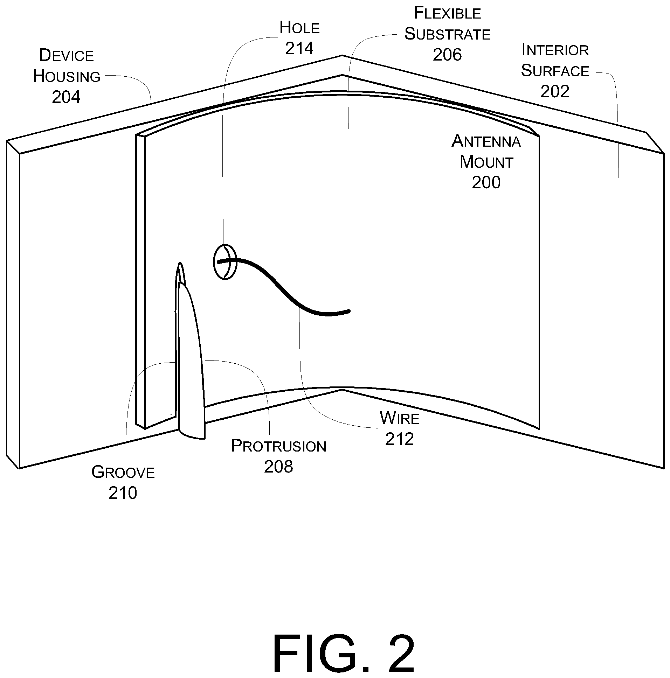

FIG. 2 illustrates an example antenna mount 200 disposed on an interior surface 202 of a device housing 204. According to various implementations, the antenna mount 200 includes a flexible substrate 206. In some cases, the flexible substrate 206 conforms to the interior surface 202 of the device housing 204. For example, the flexible substrate 206 conforms to a corner of the device housing 204. In some cases, the flexible substrate is a polymer, such as polycarbonate, polyethylene, polypropylene, acrylic, or another polymer. The thickness of the flexible substrate is, according to some implementations, between 0.1 mm and 3 mm.

In some cases, a protrusion 208 in the device housing 204 is configured to fit into a groove 210 through the flexible substrate 206. The coupling of the protrusion 208 and the groove 210 stabilizes the position of the flexible substrate 206 on the device housing 204. The protrusion 208 is, in some examples, is a rib, a notch, a latch, or any other structure that extends from the interior surface 202. In some implementations, an antenna (not shown) is disposed between the antenna mount 200 and the interior surface 202 of the device housing 204. A wire 212 connected to the antenna is routed through a hole 214 through the flexible substrate 206. An edge of the hole 214 includes the electrically insulative material.

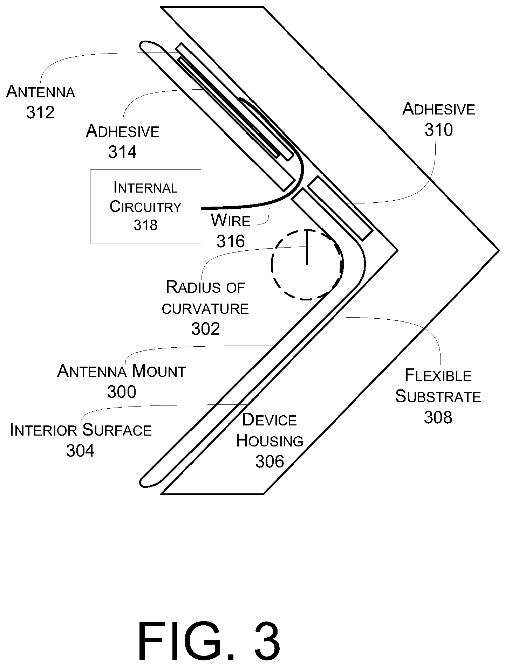

FIG. 3 illustrates an example antenna mount 300 bending at a radius of curvature 302. The antenna mount 300 is, for example, disposed on an interior surface 304 of a device housing 306. The antenna mount 300 includes a flexible substrate 308 bent at a radius of curvature 302. In some examples, the flexible substrate 308 bends at a radius of curvature 302 of 1 centimeter (cm) to conform to a corner of the device housing 306. In some cases, the radius of curvature 302 is less than 1 cm. For example, the radius of curvature can be 300 millimeters (mm). The flexible substrate 308, in some cases, can bend at a smaller radius of curvature 302 than required to conform to the device housing 306. In some cases, the antenna mount 300 is attached to the device housing 306 with an adhesive 310. The antenna mount may be removable from the device housing 306.

An antenna 312 is disposed on the antenna mount 300. According to various embodiments, the antenna 312 is attached to the antenna mount with an adhesive 314, a magnet, or a cutout. The adhesive 314, in some cases, includes at least one of double-sided tape, glue, or flat adhesive pads. The antenna 312 may be removable from the antenna mount 300. A wire 316 connected to the antenna 312 is connected to the internal circuitry 318 contained within the device housing 306.

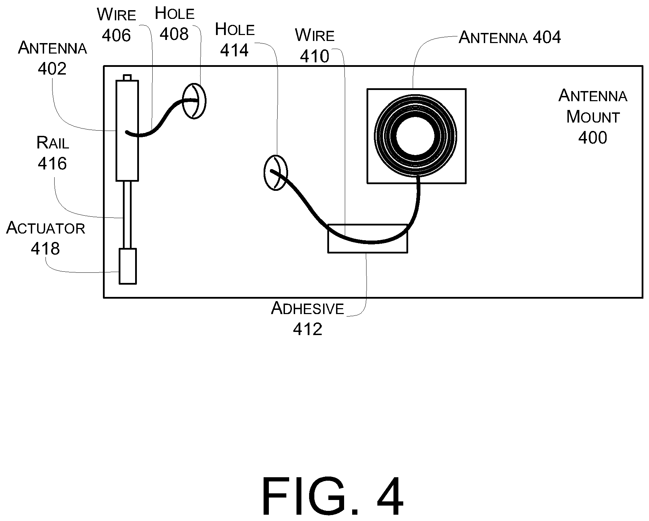

FIG. 4 illustrates an example antenna mount 400 with antenna diversity and dynamic antenna positioning. According to various implementations, the antenna mount 400 is attached to antenna 402 and antenna 404. The antenna 402, in various examples, is a flexible antenna. In some cases, the antenna 404 is a wireless charging coil. According to various examples, the antenna mount enables antenna diversity, including spatial diversity, polarization diversity, frequency diversity, pattern diversity, directional diversity, time diversity, and transmit-receive diversity. In some examples, the antenna 402 is positioned at a distance from antenna 404 for spatial diversity.

In some cases, a wire 406 connected to the antenna 402 is routed through a hole 408 through the antenna mount 400. In some cases, a wire 410 connected to the antenna 404 is attached to the antenna mount 400 with an adhesive 412, such as double-sided tape, a magnet, or a cutout. For example, the wire 410 may be attached the antenna mount 400 with the adhesive 412 and routed through a hole 414 through the antenna mount 400.

In various examples, the antenna 402 attached to a rail 416 on the antenna mount 400 to enable dynamic positioning of the antenna 402. The antenna 402 attaches to the rail 416 using a hook, a roller, a sliding block, a rack and pinion, a linear bearing, or another sliding mechanism. The rail 416 is coupled to an actuator 418 that controls the position of the antenna 402 along the rail 416. The actuator 418 is, in some examples, a servo motor, a magnetic actuator, or a shape memory alloy actuator. In some instances, the actuator 418 is controlled externally.

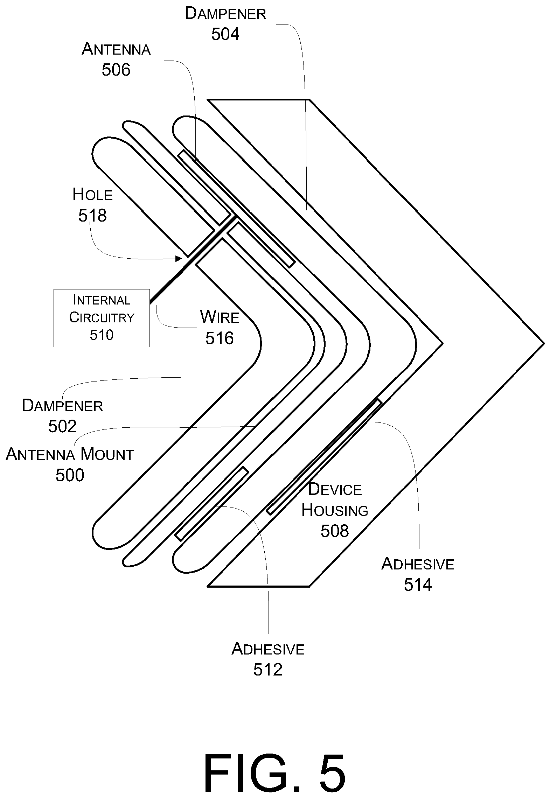

FIG. 5 illustrates an example antenna mount 500 attached to a dampener 502 and a dampener 504. According to some implementations, the dampeners 502 and 504 reduce noise or physical vibrations that may affect signal transmission by the antenna 506. A dampener (e.g., dampener 502 or dampener 504) is, in various examples, disposed between the antenna mount 500 and the device housing 508 or between the antenna mount 500 and the internal circuitry 510. The antenna mount 500 is, in some cases, attached to one of the dampeners 502 and 504. In some cases, the antenna mount 500 is attached to dampeners 502 and 504. The antenna mount 500 may be attached to a dampener (e.g., dampener 502 or dampener 504) with an adhesive 512. The dampeners 502 and 504 are, in various examples, a foam or a spray foam. The dampener 504, in some cases, is attached to the device housing 508 or other device component with an adhesive 514. A wire 516 connected to the antenna 520 is routed through a hole 518 through the dampener 502 to the internal circuitry 510.

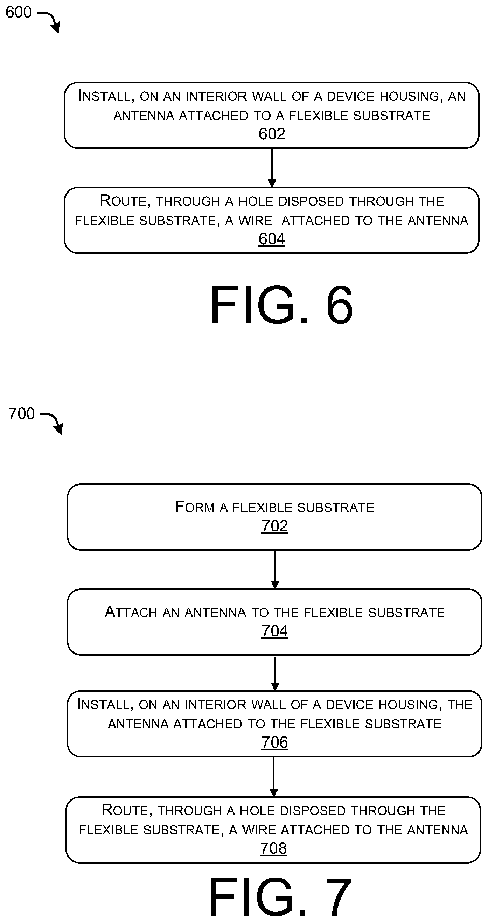

FIG. 6 illustrates an example process 600 for integrating an antenna into a device. The process 600 is performed by a trained person (e.g., a technician, an engineer, or the like) or by specialized equipment. The device may be an existing device, or a device in production. The device is, in some cases, a medical device, such as the defibrillator 102 discussed above with reference to FIG. 1.

At 602, an antenna mount including a flexible substrate is installed onto a housing of the device. The flexible substrate is attached to an antenna. The flexible substrate is, according to various examples, disposed on the interior surface of the housing of the device. The flexible substrate can be installed onto the housing of an existing device or, in some cases, onto the housing of a device in production. In some cases, the flexible substrate is attached to the housing with an adhesive. In some cases, the flexible substrate is held in place by groove(s) through the flexible substrate. The groove(s) are configured to fit around protrusion(s) in the housing. When the protrusion(s) are positioned in the groove(s), the position of the flexible substrate is stabilized.

At 604, a wire connected to the antenna is routed through a hole through the flexible substrate. The wire can, according to some examples, connect to the internal circuitry of the device. In some examples, the wire enables the antenna to transmit communication signals indicative of data generated by the internal circuitry.

FIG. 7 illustrates an example process 700 for manufacturing an antenna mount and integrating an antenna into a device. The process 700 is performed by an entity, which may include a device . The device may be an existing device, or a device in production. The device is, in some cases, a medical device, such as the defibrillator 102 discussed above with reference to FIG. 1.

At 702, the flexible substrate is formed using a cutting process. For example, the flexible substrate can be formed by laser cutting, waterjet cutting, die cutting, or stamping. In some cases, a shape of the flexible substrate is based on a housing of the device. A height of the flexible substrate, for instance, is equal to or less than a height of the housing. In some cases, a hole is formed through the flexible substrate using a cutting process. A position of the hole is, for instance, based on a position of the antenna or a position of internal circuitry in the device. In some cases, a groove is formed through the flexible substrate using a cutting process. A position of the groove is, for instance, based on a position of a protrusion in the housing.

At 704, the antenna is attached to the flexible substrate. The position of the antenna is, in some cases, determined to improve signal transmission by the antenna. For example, the position of the antenna may be at a distance from the internal circuitry in the device. The antenna, in some cases, is positioned perpendicular to a PCB in the device. In some instances, the antenna is attached to the flexible substrate with an adhesive, a magnet, or a cutout.

In some cases, a rail is attached to the antenna mount and the antenna is attached to the rail. The antenna attached to the rail using a hook, a roller, a sliding block, a rack and pinion, a linear bearing, or another sliding mechanism. The rail is coupled to an actuator that controls the position of the antenna along the rail. The actuator is, in some examples, a servo motor, a magnetic actuator, or a shape memory alloy actuator.

At 706, the flexible substrate is installed onto the housing of the device. The flexible substrate is, according to various examples, disposed on the interior surface of the housing of the device. The flexible substrate can be installed onto the housing of an existing device or, in some cases, onto the housing of a device in production. In some cases, the flexible substrate is attached to the housing with an adhesive. In some cases, the flexible substrate is held in place by groove(s) through the flexible substrate. The groove(s) are configured to fit around protrusion(s) in the housing. When the protrusion(s) are positioned in the groove(s), the position of the flexible substrate is stabilized.

At 708, a wire connected to the antenna is routed through the hole through the flexible substrate. The wire can, according to some examples, connect to the internal circuitry of the device. In some examples, the wire enables the antenna to transmit communication signals indicative of data generated by the internal circuitry.

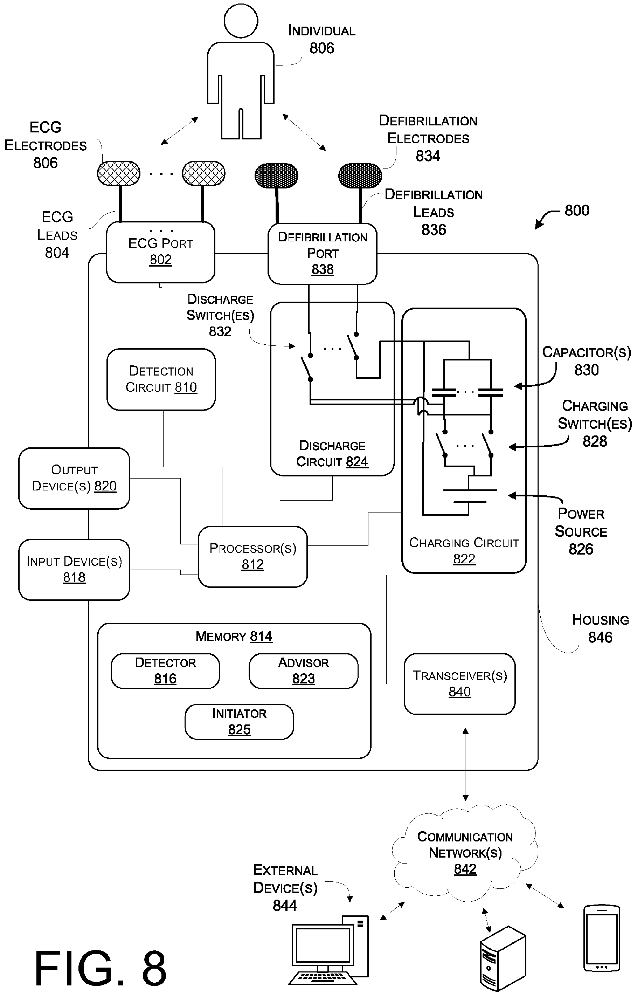

FIG. 8 illustrates an example of an external defibrillator 800 configured to perform various functions described herein. For example, the external defibrillator 800 is the defibrillator 102 described above with reference to FIG. 1.

The external defibrillator 800 includes an electrocardiogram (ECG) port 802 connected to multiple ECG leads 804. In some cases, the ECG leads 804 are removeable from the ECG port 802. For instance, the ECG leads 804 are plugged into the ECG port 802. The ECG leads 804 are connected to ECG electrodes 806, respectively. In various implementations, the ECG electrodes 806 are disposed on different locations on an individual 808. A detection circuit 810 is configured to detect relative voltages between the ECG electrodes 806. These voltages are indicative of the electrical activity of the heart of the individual 808.

In various implementations, the ECG electrodes 806 are in contact with the different locations on the skin of the individual 808. In some examples, a first one of the ECG electrodes 806 is placed on the skin between the heart and right arm of the individual 808, a second one of the ECG electrodes 806 is placed on the skin between the heart and left arm of the individual 808, and a third one of the ECG electrodes 806 is placed on the skin between the heart and a leg (either the left leg or the right leg) of the individual 808. In these examples, the detection circuit 810 is configured to measure the relative voltages between the first, second, and third ECG electrodes 806. Respective pairings of the ECG electrodes 806 are referred to as "leads," and the voltages between the pairs of ECG electrodes 806 are known as "lead voltages." In some examples, more than three ECG electrodes 806 are included, such that 5-lead or 12-lead ECG signals are detected by the detection circuit 810.

The detection circuit 810 includes at least one analog circuit, at least one digital circuit, or a combination thereof. The detection circuit 810 receives the analog electrical signals from the ECG electrodes 806, via the ECG port 802 and the ECG leads 804. In some cases, the detection circuit 810 includes one or more analog filters configured to filter noise and/or artifact from the electrical signals. The detection circuit 810 includes an analog-to-digital (ADC) in various examples. The detection circuit 810 generates a digital signal indicative of the analog electrical signals from the ECG electrodes 806. This digital signal can be referred to as an "ECG signal" or an "ECG."

In some cases, the detection circuit 810 further detects an electrical impedance between at least one pair of the ECG electrodes 806. For example, the detection circuit 810 includes, or otherwise controls, a power source that applies a known voltage (or current) across a pair of the ECG electrodes 806 and detects a resultant current (or voltage) between the pair of the ECG electrodes 806. The impedance is generated based on the applied signal (voltage or current) and the resultant signal (current or voltage). In various cases, the impedance corresponds to respiration of the individual 808, chest compressions performed on the individual 808, and other physiological states of the individual 808. In various examples, the detection circuit 810 includes one or more analog filters configured to filter noise and/or artifact from the resultant signal. The detection circuit 810 generates a digital signal indicative of the impedance using an ADC. This digital signal can be referred to as an "impedance signal" or an "impedance."

The detection circuit 810 provides the ECG signal and/or the impedance signal to one or more processors 812 in the external defibrillator 800. In some implementations, the processor(s) 812 includes a central processing unit (CPU), a graphics processing unit (GPU), both CPU and GPU, or other processing unit or component known in the art.

The processor(s) 812 is operably connected to memory 814. In various implementations, the memory 814 is volatile (such as random access memory (RAM)), nonvolatile (such as read only memory (ROM), flash memory, etc.) or some combination of the two. The memory 814 stores instructions that, when executed by the processor(s) 812, causes the processor(s) 812 to perform various operations. In various examples, the memory 814 stores methods, threads, processes, applications, objects, modules, any other sort of executable instruction, or a combination thereof. In some cases, the memory 814 stores files, databases, or a combination thereof. In some examples, the memory 814 includes, but is not limited to, RAM, ROM, electrically erasable programmable read-only memory (EEPROM), flash memory, or any other memory technology. In some examples, the memory 814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 812 and/or the external defibrillator 800. In some cases, the memory 814 at least temporarily stores the ECG signal and/or the impedance signal.

In various examples, the memory 814 includes a detector 816, which causes the processor(s) 812 to determine, based on the ECG signal and/or the impedance signal, whether the individual 808 is exhibiting a particular heart rhythm. For instance, the processor(s) 812 determines whether the individual 808 is experiencing a shockable rhythm that is treatable by defibrillation. Examples of shockable rhythms include ventricular fibrillation (VF) and pulseless ventricular tachycardia (VT). In some examples, the processor(s) 812 determines whether any of a variety of different rhythms (e.g., asystole, sinus rhythm, atrial fibrillation (AF), etc.) are present in the ECG signal.

The processor(s) 812 is operably connected to one or more input devices 818 and one or more output devices 820. Collectively, the input device(s) 818 and the output device(s) 820 function as an interface between a user and the defibrillator 800. The input device(s) 818 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 820 includes at least one of a display, a speaker, a haptic output device, a printer, or any combination thereof. In various examples, the processor(s) 812 causes a display among the input device(s) 818 to visually output a waveform of the ECG signal and/or the impedance signal. In some implementations, the input device(s) 818 includes one or more touch sensors, the output device(s) 820 includes a display screen, and the touch sensor(s) are integrated with the display screen. Thus, in some cases, the external defibrillator 800 includes a touchscreen configured to receive user input signal(s) and visually output physiological parameters, such as the ECG signal and/or the impedance signal.

In some examples, the memory 814 includes an advisor 823, which, when executed by the processor(s) 812, causes the processor(s) 812 to generate advice and/or control the output device(s) 820 to output the advice to a user (e.g., a rescuer). In some examples, the processor(s) 812 provides, or causes the output device(s) 820 to provide, an instruction to perform CPR on the individual 808. In some cases, the processor(s) 812 evaluates, based on the ECG signal, the impedance signal, or other physiological parameters, CPR being performed on the individual 808 and causes the output device(s) 820 to provide feedback about the CPR in the instruction. According to some examples, the processor(s) 812, upon identifying that a shockable rhythm is present in the ECG signal, causes the output device(s) 820 to output an instruction and/or recommendation to administer a defibrillation shock to the individual 808.

The memory 814 also includes an initiator 825 which, when executed by the processor(s) 812, causes the processor(s) 812 to control other elements of the external defibrillator 800 in order to administer a defibrillation shock to the individual 808. In some examples, the processor(s) 812 executing the initiator 825 selectively causes the administration of the defibrillation shock based on determining that the individual 808 is exhibiting the shockable rhythm and/or based on an input from a user (received, e.g., by the input device(s) 818. In some cases, the processor(s) 812 causes the defibrillation shock to be output at a particular time, which is determined by the processor(s) 812 based on the ECG signal and/or the impedance signal.

The processor(s) 812 is operably connected to a charging circuit 822 and a discharge circuit 824. In various implementations, the charging circuit 822 includes a power source 826, one or more charging switches 828, and one or more capacitors 830. The power source 826 includes, for instance, a battery. The processor(s) 812 initiates a defibrillation shock by causing the power source 826 to charge at least one capacitor among the capacitor(s) 830. For example, the processor(s) 812 activates at least one of the charging switch(es) 828 in the charging circuit 822 to complete a first circuit connecting the power source 826 and the capacitor to be charged. Then, the processor(s) 812 causes the discharge circuit 824 to discharge energy stored in the charged capacitor across a pair of defibrillation electrodes 834, which are in contact with the individual 808. For example, the processor(s) 812 deactivates the charging switch(es) 828 completing the first circuit between the capacitor(s) 830 and the power source 826, and activates one or more discharge switches 832 completing a second circuit connecting the charged capacitor 830 and at least a portion of the individual 808 disposed between defibrillation electrodes 834.

The energy is discharged from the defibrillation electrodes 834 in the form of a defibrillation shock. For example, the defibrillation electrodes 834 are connected to the skin of the individual 808 and located at positions on different sides of the heart of the individual 808, such that the defibrillation shock is applied across the heart of the individual 808. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 832 are controlled by the processor(s) 812, for example. In various implementations, the defibrillation electrodes 834 are connected to defibrillation leads 836. The defibrillation leads 836 are connected to a defibrillation port 838, in implementations. According to various examples, the defibrillation leads 836 are removable from the defibrillation port 838. For example, the defibrillation leads 836 are plugged into the defibrillation port 838.

In various implementations, the processor(s) 812 is operably connected to one or more transceivers 840 that transmit and/or receive data over one or more communication networks 842. For example, the transceiver(s) 840 includes a network interface card (NIC), a network adapter, a local area network (LAN) adapter, or a physical, virtual, or logical address to connect to the various external devices and/or systems. In various examples, the transceiver(s) 840 includes any sort of wireless transceivers capable of engaging in wireless communication (e.g., radio frequency (RF) communication). For example, the communication network(s) 842 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 840 includes other wireless modems, such as a modem for engaging in WI-FI^{®}, WIGIG^{®}, WIMAX^{®}, BLUETOOTH^{®}, or infrared communication over the communication network(s) 842.

The defibrillator 800 is configured to transmit and/or receive data (e.g., ECG data, impedance data, data indicative of one or more detected heart rhythms of the individual 808, data indicative of one or more defibrillation shocks administered to the individual 808, etc.) with one or more external devices 844 via the communication network(s) 842. The external devices 844 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 842. In some examples, the external device(s) 844 is located remotely from the defibrillator 800, such as at a remote clinical environment (e.g., a hospital). According to various implementations, the processor(s) 812 causes the transceiver(s) 840 to transmit data to the external device(s) 844. In some cases, the transceiver(s) 840 receives data from the external device(s) 844 and the transceiver(s) 840 provide the received data to the processor(s) 812 for further analysis.

In various implementations, the external defibrillator 800 also includes a housing 846 that at least partially encloses other elements of the external defibrillator 800. For example, the housing 846 encloses the detection circuit 810, the processor(s) 812, the memory 814, the charging circuit 822, the transceiver(s) 840, or any combination thereof. In some cases, the input device(s) 818 and output device(s) 820 extend from an interior space at least partially surrounded by the housing 846 through a wall of the housing 846. In various examples, the housing 846 acts as a barrier to moisture, electrical interference, and/or dust, thereby protecting various components in the external defibrillator 800 from damage. In various examples, the transceiver(s) 840 are physically attached to the housing 846 of the defibrillator 800 via an antenna mount. For instance, the detection circuit 810, the processor(s) 812, the memory 814, the charging circuit 822, or any combination thereof, may be internal circuitry that is communicatively coupled to the transceiver(s) 840 via at least one wire extending through at least one hole in the antenna mount.

In some implementations, the external defibrillator 800 is an automated external defibrillator (AED) operated by an untrained user (e.g., a bystander, layperson, etc.) and can be operated in an automatic mode. In automatic mode, the processor(s) 812 automatically identifies a rhythm in the ECG signal, makes a decision whether to administer a defibrillation shock, charges the capacitor(s) 830, discharges the capacitor(s) 830, or any combination thereof. In some cases, the processor(s) 812 controls the output device(s) 820 to output (e.g., display) a simplified user interface to the untrained user. For example, the processor(s) 812 refrains from causing the output device(s) 820 to display a waveform of the ECG signal and/or the impedance signal to the untrained user, in order to simplify operation of the external defibrillator 800.

In some examples, the external defibrillator 800 is a monitor-defibrillator utilized by a trained user (e.g., a clinician, an emergency responder, etc.) and can be operated in a manual mode or the automatic mode. When the external defibrillator 800 operates in manual mode, the processor(s) 812 cause the output device(s) 820 to display a variety of information that may be relevant to the trained user, such as waveforms indicating the ECG data and/or impedance data, notifications about detected heart rhythms, and the like. In some examples, the external defibrillator 800 is an AED utilized by a trained user.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Moreover, any combination of the above-described elements in all possible variations thereof is encompassed by implementations of the disclosure unless otherwise indicated herein or otherwise clearly contradicted by context.

## Claims

1. A device (102), comprising:
a housing (116, 204, 306, 508, 846);
an antenna mount (118, 200, 300, 400, 500) disposed on an interior surface (120, 202, 304) of the housing and comprising a material that is flexible and electrically insulative, wherein a hole (136, 214, 414,518) extends through the antenna mount;
a flexible antenna (126, 128, 312,404, 506) disposed between the interior surface of the housing and the antenna mount; **characterized in that**
the device comprises a defibrillator (800), comprising
a measurement circuit (114, 318, 510, 810, 812) configured to detect an electrocardiogram (ECG) of a patient;
a wire (128, 212, 316, 412, 516) disposed in the hole through the antenna mount, the wire being connected to the flexible antenna and the measurement circuit;
a treatment circuit (822, 824) configured to output an electrical shock to the patient; and
a processor (812) configured to:
determine, by analyzing the ECG, that the patient is exhibiting ventricular fibrillation (VF);
in response to determining that the patient is exhibiting VF, causing the treatment circuit to output the electrical shock to the patient; and
cause the flexible antenna to transmit an electromagnetic (EM) signal indicating the ECG, the VF, and the electrical shock.

2. The device (102) of claim 1, wherein the material comprises polycarbonate, polyethylene, polypropylene, acrylic, or aramid paper, optionally
wherein the antenna mount (118, 200, 300, 400, 500) has a thickness of between about 0.1 millimeters (mm) and about 3 mm, optionally
wherein an edge of the hole (136, 214, 414,518) through the antenna mount comprises the material that is flexible and electrically insulative.

3. The device (102) according to any of the preceding claims, the interior surface (120, 202, 304) comprising a protrusion (208), the antenna mount (118, 200, 300, 400, 500) further comprising:
a groove (210) in the flexible substrate (206), the protrusion being configured to fit in the groove, optionally
wherein the device housing (116, 204, 306, 508, 846) is part of a defibrillator (800) or mechanical chest compression device, optionally
wherein the electrically insulative material comprises a polymer or a foam, optionally
the antenna mount (118, 200, 300, 400, 500) further comprising:
a dampener (502, 504) disposed on the flexible substrate,
wherein the dampener comprises a foam.

4. The device (102) according to any of the preceding claims, further comprising:
a rail (416) configured to attach to an antenna (402); and
an actuator (418) coupled to the rail (416) and configured to move the antenna (402) along the rail, wherein the actuator comprises a servo motor, a magnetic actuator, or a shape memory alloy actuator.

5. A method of manufacturing a medical device, comprising:
installing an antenna (126, 128, 312,404, 506) between an antenna mount (118, 200, 300, 400, 500) and an interior wall (120, 202, 304) of a device housing (116, 204, 306, 508, 846), the antenna mount comprising a flexible substrate configured to conform to the interior wall of the device housing (116, 204, 306, 508, 846), the flexible substrate comprising an electrically insulative material;
routing a wire (128, 212, 316, 412, 516) from the antenna through a hole (136, 214, 414,518) in the antenna mount; and
connecting the wire to a circuit (114, 318, 510, 810, 810, 812) of the medical device.

6. The method of claim 5, wherein installing the antenna (126, 128, 312,404, 506) comprises attaching the antenna to a rail (416) disposed on the antenna mount (400).

7. The method of claim 6, further comprising:
installing an actuator (418) configured to move the antenna (402) along the rail (416), the actuator comprising a servo motor, a magnetic actuator, or a shape memory alloy actuator.

8. The method of claim 5, wherein the antenna (126, 128, 312,404, 506) comprises a flexible antenna, a communication antenna, or a wireless charging antenna.

9. The method of claim5, further comprising:
securing the flexible substrate (206) on the interior wall (202) of the device housing by positioning a protrusion (208) on the interior wall through a groove (210) in the antenna mount (200).

10. The method of claim 5, wherein the antenna is a first antenna, further comprising:
installing a second antenna between the antenna mount (118, 200, 300, 400, 500) and the interior wall (120, 202, 304) of the device housing (116, 204, 306, 508, 846).

11. The method of claim 5, further comprising:
forming the flexible substrate configured to conform to the interior wall (120, 202, 304) of the device housing (116, 204, 306, 508, 846); and
attaching the antenna (126, 128, 312,404, 506) to the flexible substrate with an adhesive, a magnet, or a cutout.

12. The method of claim 11, wherein forming the flexible substrate comprises a laser cutting process, a die cutting process, a waterjet cutting process, or a stamping process.

13. The method of claim5, further comprising:
attaching a dampener (502, 504) to the flexible substrate.

## Patentansprüche

1. Vorrichtung (102), umfassend:
ein Gehäuse (116, 204, 306, 508, 846);
eine Antennenhalterung (118, 200, 300, 400, 500), die an einer Innenfläche (120, 202, 304) des Gehäuses angeordnet ist und ein Material umfasst, das flexibel und elektrisch isolierend ist, wobei sich ein Loch (136, 214, 414, 518) durch die Antennenhalterung erstreckt;
eine flexible Antenne (126, 128, 312, 404, 506), die zwischen der Innenfläche des Gehäuses und der Antennenhalterung angeordnet ist; **dadurch gekennzeichnet, dass**
die Vorrichtung einen Defibrillator (800) umfasst, der umfasst:
eine Messschaltung (114, 318, 510, 810, 812), die dazu ausgelegt ist, ein Elektrokardiogramm (EKG) eines Patienten zu erfassen;
einen Draht (128, 212, 316, 412, 516), der in dem Loch durch die Antennenhalterung angeordnet ist, wobei der Draht mit der flexiblen Antenne und der Messschaltung verbunden ist;
eine Behandlungsschaltung (822, 824), die so ausgelegt ist, dass sie einen elektrischen Schock an den Patienten abgibt; und
einen Prozessor (812), der konfiguriert ist zum:
durch Analysieren des EKG bestimmt, dass der Patient Kammerflimmern (VF) aufweist;
in Reaktion auf das Bestimmen, dass der Patient VF aufweist, die Behandlungsschaltung dazu veranlasst, den elektrischen Schock an den Patienten abzugeben; und
die flexible Antenne dazu veranlasst, ein elektromagnetisches (EM-) Signal zu senden, das das EKG, das VF und den elektrischen Schock anzeigt.

2. Vorrichtung (102) nach Anspruch 1, wobei das Material Polycarbonat, Polyethylen, Polypropylen, Acryl oder Aramidpapier umfasst, wahlweise
wobei die Antennenhalterung (118, 200, 300, 400, 500) eine Dicke zwischen etwa 0,1 Millimeter (mm) und etwa 3 mm aufweist, wahlweise
wobei ein Rand des Lochs (136, 214, 414, 518) durch die Antennenhalterung das Material umfasst, das flexibel und elektrisch isolierend ist.

3. Vorrichtung (102) nach einem der vorhergehenden Ansprüche, wobei die Innenfläche (120, 202, 304) einen Vorsprung (208) umfasst, wobei die Antennenhalterung (118, 200, 300, 400, 500) ferner umfasst:
eine Nut (210) in dem flexiblen Substrat (206), wobei der Vorsprung so ausgelegt ist, dass er in die Nut passt, wahlweise
wobei das Vorrichtungsgehäuse (116, 204, 306, 508, 846) Teil eines Defibrillators (800) oder einer mechanischen Thoraxkompressionsvorrichtung ist, wahlweise wobei das elektrisch isolierende Material ein Polymer oder einen Schaumstoff umfasst, wahlweise wobei die Antennenhalterung (118, 200, 300, 400, 500) ferner umfasst:
einen Dämpfer (502, 504), der auf dem flexiblen Substrat angeordnet ist,
wobei der Dämpfer einen Schaumstoff umfasst.

4. Vorrichtung (102) nach einem der vorhergehenden Ansprüche, ferner umfassend:
eine Schiene (416), die zur Anbringung an einer Antenne (402) ausgelegt ist; und
einen Aktuator (418), der mit der Schiene (416) gekoppelt und dazu ausgelegt ist, die Antenne (402) entlang der Schiene zu bewegen, wobei der Aktuator einen Servomotor, einen magnetischen Aktuator oder einen Aktuator aus einer Formgedächtnislegierung umfasst.

5. Verfahren zur Herstellung einer medizinischen Vorrichtung, umfassend:
Installieren einer Antenne (126, 128, 312, 404, 506) zwischen einer Antennenhalterung (118, 200, 300, 400, 500) und einer Innenwand (120, 202, 304) eines Vorrichtungsgehäuses (116, 204, 306, 508, 846), wobei die Antennenhalterung ein flexibles Substrat umfasst, das so ausgelegt ist, dass es sich an die Innenwand des Vorrichtungsgehäuses (116, 204, 306, 508, 846) anpasst, und wobei das flexible Substrat ein elektrisch isolierendes Material umfasst;
Verlegen eines Kabels (128, 212, 316, 412, 516) von der Antenne durch ein Loch (136, 214, 414, 518) in der Antennenhalterung; und
Verbinden des Kabels mit einer Schaltung (114, 318, 510, 810, 810, 812) der medizinischen Vorrichtung.

6. Verfahren nach Anspruch 5, wobei das Installieren der Antenne (126, 128, 312, 404, 506) Anbringen der Antenne an einer Schiene (416) umfasst, die an der Antennenhalterung (400) angeordnet ist.

7. Verfahren nach Anspruch 6, ferner umfassend:
Installieren eines Aktuators (418), der dazu ausgelegt ist, die Antenne (402) entlang der Schiene (416) zu bewegen, wobei der Aktuator einen Servomotor, einen magnetischen Aktuator oder einen Aktuator aus einer Formgedächtnislegierung umfasst.

8. Verfahren nach Anspruch 5, wobei die Antenne (126, 128, 312, 404, 506) eine flexible Antenne, eine Kommunikationsantenne oder eine Antenne für drahtloses Laden umfasst.

9. Verfahren nach Anspruch 5, ferner umfassend:
Sichern des flexiblen Substrats (206) an der Innenwand (202) des Vorrichtungsgehäuses, indem ein Vorsprung (208) an der Innenwand durch eine Nut (210) in der Antennenhalterung (200) geführt wird.

10. Verfahren nach Anspruch 5, wobei es sich bei der Antenne um eine erste Antenne handelt, ferner umfassend:
Installieren einer zweiten Antenne zwischen der Antennenhalterung (118, 200, 300, 400, 500) und der Innenwand (120, 202, 304) des Vorrichtungsgehäuses (116, 204, 306, 508, 846).

11. Verfahren nach Anspruch 5, ferner umfassend:
Ausbilden des flexiblen Substrats, das so ausgelegt ist, dass es sich an die Innenwand (120, 202, 304) des Vorrichtungsgehäuses (116, 204, 306, 508, 846) anpasst; und
Anbringen der Antenne (126, 128, 312, 404, 506) an dem flexiblen
Substrat mittels eines Klebstoffs, eines Magneten oder einer Aussparung.

12. Verfahren nach Anspruch 11, wobei das Herstellen des flexiblen Substrats einen Laserschneidprozess, einen Stanzprozess, einen Wasserstrahlschneidprozess oder einen Prägeprozess umfasst.

13. Verfahren nach Anspruch 5, ferner umfassend:
Anbringen eines Dämpfers (502, 504) an dem flexiblen Substrat.

## Revendications

1. Dispositif (102), comprenant :
un boîtier (116, 204, 306, 508, 846) ;
un support d'antenne (118, 200, 300, 400, 500) disposé sur une surface intérieure (120, 202, 304) du boîtier et comprenant un matériau qui est flexible et isolant électriquement, dans lequel un trou (136, 214, 414, 518) s'étend à travers le support d'antenne ;
une antenne flexible (126, 128, 312, 404, 506) disposée entre la surface intérieure du boîtier et le support d'antenne ; **caractérisé en ce que**
le dispositif comprend un défibrillateur (800), comprenant
un circuit de mesure (114, 318, 510, 810, 812) configuré pour détecter un électrocardiogramme (ECG) d'un patient ;
un fil (128, 212, 316, 412, 516) disposé dans le trou à travers le support d'antenne, le fil étant connecté à l'antenne flexible et au circuit de mesure ;
un circuit de traitement (822, 824) configuré pour administrer un choc électrique au patient ; et
un processeur (812) configuré pour :
déterminer, en analysant l'ECG, que le patient présente une fibrillation ventriculaire (FV) ;
en réponse à la détermination que le patient présente une FV, amener le circuit de traitement à administrer le choc électrique au patient ; et
amener l'antenne flexible à transmette un signal électromagnétique (EM) indiquant l'ECG, la FV et le choc électrique.

2. Dispositif (102) selon la revendication 1, dans lequel le matériau comprend du polycarbonate, du polyéthylène, du polypropylène, de l'acrylique ou du papier aramide, facultativement
dans lequel le support d'antenne (118, 200, 300, 400, 500) a une épaisseur entre environ 0,1 millimètre (mm) et environ 3 mm, facultativement
dans lequel un bord du trou (136, 214, 414, 518) à travers le support d'antenne comprend le matériau qui est flexible et électriquement isolant.

3. Dispositif (102) selon l'une quelconque des revendications précédentes, la surface intérieure (120, 202, 304) comprenant une saillie (208), le support d'antenne (118, 200, 300, 400, 500) comprenant en outre :
une rainure (210) dans le substrat flexible (206), la saillie étant configurée pour s'ajuster dans la rainure, facultativement
dans lequel le boîtier de dispositif (116, 204, 306, 508, 846) fait partie d'un défibrillateur (800) ou d'un dispositif de compression thoracique mécanique, facultativement
dans lequel le matériau électriquement isolant comprend un polymère ou une mousse, facultativement le support d'antenne (118, 200, 300, 400, 500) comprenant en outre :
un amortisseur (502, 504) disposé sur le substrat flexible,
dans lequel l'amortisseur comprend une mousse.

4. Dispositif (102) selon l'une quelconque des revendications précédentes, comprenant en outre :
un rail (416) configuré pour se fixer à une antenne (402) ; et
un actionneur (418) accouplé au rail (416) et configuré pour déplacer l'antenne (402) le long du rail, l'actionneur comprenant un servomoteur, un actionneur magnétique ou un actionneur en alliage à mémoire de forme.

5. Procédé de fabrication d'un dispositif médical, comprenant :
l'installation d'une antenne (126, 128, 312,404, 506) entre un support d'antenne (118, 200, 300, 400, 500) et une paroi intérieure (120, 202, 304) d'un boîtier de dispositif (116, 204, 306, 508, 846), le support d'antenne comprenant un substrat flexible configuré pour épouser la forme de la paroi intérieure du boîtier de dispositif (116, 204, 306, 508, 846), le substrat flexible comprenant un matériau électriquement isolant ; acheminer un fil (128, 212, 316, 412, 516) à partir de l'antenne à travers un trou (136, 214, 414, 518) dans le support d'antenne ; et
connecter le fil à un circuit (114, 318, 510, 810, 810, 812) du dispositif médical.

6. Procédé selon la revendication 5, dans lequel l'installation de l'antenne (126, 128, 312, 404, 506) comprend la fixation de l'antenne à un rail (416) disposé sur le support d'antenne (400).

7. Procédé selon la revendication 6, comprenant en outre :
l'installation d'un actionneur (418) configuré pour déplacer l'antenne (402) le long du rail (416), l'actionneur comprenant un servomoteur, un actionneur magnétique ou un actionneur en alliage à mémoire de forme.

8. Procédé selon la revendication 5, dans lequel l'antenne (126, 128, 312, 404, 506) comprend une antenne flexible, une antenne de communication ou une antenne de charge sans fil.

9. Procédé selon la revendication 5, comprenant en outre :
l'assujettissement du substrat flexible (206) sur la paroi intérieure (202) du boîtier de dispositif en positionnant une saillie (208) sur la paroi intérieure à travers une rainure (210) dans le support d'antenne (200).

10. Procédé selon la revendication 5, dans lequel l'antenne est une première antenne, comprenant en outre : l'installation d'une seconde antenne entre le support d'antenne (118, 200, 300, 400, 500) et la paroi intérieure (120, 202, 304) du boîtier de dispositif (116, 204, 306, 508, 846).

11. Procédé selon la revendication 5, comprenant en outre :
la formation du substrat flexible configuré pour épouser la forme de la paroi intérieure (120, 202, 304) du boîtier de dispositif (116, 204, 306, 508, 846) ; et
la fixation de l'antenne (126, 128, 312, 404, 506) au substrat flexible avec un adhésif, un aimant ou une découpe.

12. Procédé selon la revendication 11, dans lequel la formation du substrat flexible comprend un processus de découpe au laser, un processus de découpe à l'emporte-pièce, un processus de découpe au jet d'eau ou un processus d'estampage.

13. Procédé selon la revendication 5, comprenant en outre :
la fixation d'un amortisseur (502, 504) au substrat flexible.
